# EUROPEAN PATENT APPLICATION

(11) **EP 3 903 590 A1**
(43) Date of publication of application: **03.11.2021**
(21) Application number: 21171701.2
(22) Date of filing: 30.04.2021
(51) Int. Cl.: A23B 7/152, A01N 27/00

(54) **SYSTEM AND METHOD FOR RECONDITIONING POTATOES**

(30) Priority: 01.05.2020 GB 202006444
(71) Applicant: Restrain Company Limited, Business Park Lynch Wood Lynch Wood Peterborough PE2 6FT (GB)
(72) Inventor: Garos, Dirk, Peterborough, PE2 6FT (GB); O'Connor, Paul, Peterborough, PE2 6FT (GB); Armstrong, Angus, Peterborough, PE2 6FT (GB); Coleman, Paul, Peterborough, PE2 6FT (GB)
(74) Representative: Williams Powell

(57) **Abstract**

A method of reconditioning potatoes are disclosed. Over a time period of 1 week or more, potatoes are stored in a controlled environment having a temperature of >=10°C and an atmosphere comprising ethylene at a concentration greater than 0.1 ppm. A corresponding system is also disclosed.

## Description

### Field of the Invention

The present invention relates to a method and system for controlling reconditioning of potatoes.

### Background to the Invention

Harvested potatoes will exhibit sprout growth while stored unless preventative action is taken. Although a potato that has started to sprout is still safe to consume, the sprout itself contains solanine and other glycoalkaloids that can be toxic if ingested in sufficient quantities. Sprouts are therefore considered undesirable by retailers and consumers.

If potatoes are left to sprout, those sprouts also tend to be rubbery and difficult to remove from the potato.

Sprout suppressants can be used such as chlorpropham (CIPC), ethylene, maleic hydrazide and spearmint oil. However, pesticides and other chemicals are also considered undesirable.

Potatoes sprout at a rate primarily determined by temperature. Reducing storage temperature is, therefore, an effective way of controlling sprouting, by first prolonging dormancy and then limiting the rate of growth. To avoid use of sprout suppressants entirely, storage temperatures need to be controlled, often at temperatures of 2.5-9°C (dependent on variety and intended use).

However, potatoes held at cold temperatures will be affected by low-temperature sweetening which can adversely affect taste, texture and colour on roasting/ frying. Where potatoes are destined for frying, quality is determined by the amount of sugars present (the lower sugars being equated with higher quality) as the sugars react on cooking to produce browning (fry colour). Presence of too much sugar will cause a crop destined for frying to be rejected during quality control as it will exhibit too dark a fry colour. Similarly, a potato crop may be destined for use in starch - the sugar creation process consumes starch and reduces the potato quality (and likelihood of acceptance) for this intended use.

Reconditioning is the term used in the potato industry for the process used to reduce and/or remove the sugars in the potato. During reconditioning, stored potatoes are subjected to temperatures above 10°C. The increase in temperature causes resynthesis of starch from free sugars. The length of reconditioning depends on the level of sugars accumulated during cold storage and on the susceptibility of different potato varieties to lose sugar during this treatment.

However, reconditioning requires temperatures higher than the normal 2.5-9°C used to suppress sprouting and undermines the sprout-suppression regime.

### Statement of Invention

According to an aspect of the present invention, there is provided a method of reconditioning potatoes comprising:
over a time period of 1 week or more, storing the potatoes in a controlled environment having a temperature of >=10°C and an atmosphere comprising ethylene at a concentration greater than 0.1 ppm.

The present invention seeks to address the problems and disadvantages of existing apparatus and methods for reconditioning potatoes. In particular, it has been identified and confirmed through testing by the inventors of the present invention that controlled application of ethylene during reconditioning causes any sprouting to become brittle and easily removed during unloading the store and distribution. Application of ethylene to potatoes is understood to "stress" the potato and induce sugars. However, the inventors have identified a protocol as set out in the described method and system that avoids ethylene inducement of sugars and means that both reconditioning and ethylene application can take place concurrently and in a complementary manner. As a result, reconditioned potatoes exhibit lower and/or more brittle sprouting with reduced sugar content. This means that the reconditioned potatoes are of higher processability (quality) when considered for chip and crisp frying or when used for starch.

One embodiment is directed to a method in which potatoes are initially stored at reduced temperatures of around 6-9 Celsius followed by a reconditioning period in which the environment is warmed up 10 to 22 Celsius for 1 to 4 weeks in the presence of ethylene. Embodiments may use various schemes for managing temperature and ethylene concentration. Embodiments include an ethylene reverse slow-start from 10ppm-0.1ppm; an ethylene start from 10ppm dropping over time to 0.1ppm.

According to another aspect of the present invention, there is provided a system for controlling reconditioning of potatoes in a storage facility comprising:
a temperature sensor and a temperature controller, the temperature sensor being configured to monitor temperature of an atmosphere in the storage facility and communicate the temperature to the temperature controller, the temperature controller being configured to control temperature of the environment according to a predetermined reconditioning programme, the predetermined reconditioning programme maintaining a temperature in the atmosphere of greater than or equal to 10 degrees Celsius for at least one week;
an ethylene concentration sensor and an ethylene concentration controller, the ethylene concentration sensor being configured to detect an ethylene concentration in the atmosphere and communicate the concentration to the ethylene concentration controller, the ethylene concentration controller being configured to control ethylene concentration in the atmosphere according to a predetermined ethylene concentration programme, the predetermined ethylene concentration programme comprising a concentration of ethylene in the atmosphere during the at least one week of at least 0.1 ppm.

According to a further aspect of the present invention, there is provided a computer program that may optionally be encoded on a computer readable medium. When executed by a computer processor, the computer program is configured to execute the method as described above.

### Brief Description of the Drawings

Embodiments of the present invention will now be described, by way of example only with reference to the accompanying drawings in which:
Figure 1 is a schematic diagram of a potato reconditioning system according to an embodiment;
Figure 2 is a series of photographs showing fry colour before reconditioning; and,
Figure 3 is a series of photographs showing fry colour after reconditioning using the embodiment of Figure 1.

### Detailed Description

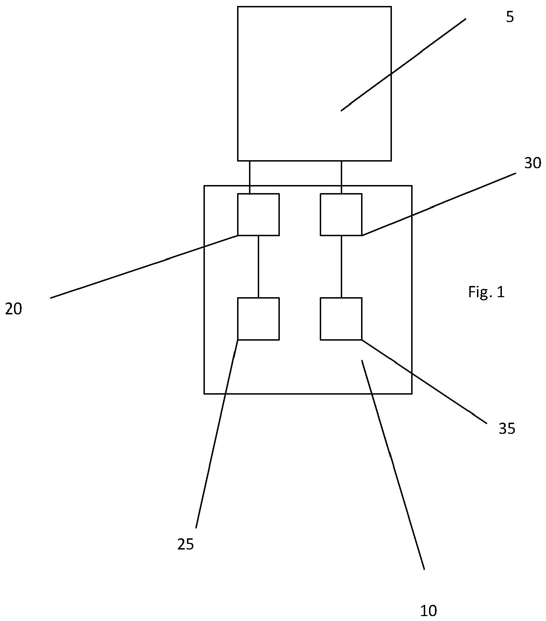

Figure 1 is a schematic diagram of a potato reconditioning system according to an embodiment.

The system 10 is installed at an above ground potato storage facility 5 (but it will be appreciated can be supplied as a separate system and retro-fitted to existing storage facilities and/or be portable and used temporarily at a facility before removal for use elsewhere, storage etc).

The system 10 includes a temperature sensor 20 and a temperature controller 25 and an ethylene concentration sensor 30 and an ethylene concentration controller 35.

The temperature sensor 20 is configured to monitor temperature of the atmosphere in the storage facility 5. It communicates the temperature to the temperature controller 25 which in turn is configured to control temperature of the environment according to a predetermined reconditioning programme. The predetermined reconditioning programme maintains a temperature in the atmosphere of greater than or equal to 10 degrees Celsius for at least one week.

The ethylene concentration sensor 30 is configured to detect an ethylene concentration in the atmosphere and communicate the concentration to the ethylene concentration controller 35. The ethylene concentration controller 35 in turn is configured to control ethylene concentration in the atmosphere according to a predetermined ethylene concentration programme. The predetermined ethylene concentration programme comprises a concentration of ethylene in the atmosphere during the at least one week of at least 0.1 ppm.

Temperature and ethylene concentration changes can be achieved in many different ways, for example via a heater for temperature and via an ethylene generator for ethylene. These could be part of the system 10, part of the facility 5 or separate systems that are triggered by the respective controllers. An example of an ethylene generator is set out in EP1985187, the content of which is incorporated herein by reference.

It will be appreciated that the reconditioning programme and the ethylene concentration programme may be configured according to many different profiles, examples of which are set out below. The programmes may be fixed or variable and may be part of the respective controllers 25, 35 or communicated from a central controller or other remote system. The temperature and ethylene concentration controllers 25, 35 preferably operate on a feedback or other control principle so as to follow the respective programme over time and accommodate external influences such as weather changes that may impact temperature etc. optionally, a control potato or a series of control potatoes in the facility 5 may be continuously or intermittently monitored for sugar content or fry colour and the programmes varied in dependence on the monitoring. This may be done manually by a technician or in an automated fashion using a probe in the potato or similar.

In one example, temperature may be set (approximately) between 10°C and 20°C and held at that temperature by the reconditioning programme. More preferably, the temperature is held at between 12 and 14 °C. Most preferably, temperature is held at 14 °C. The programme may be performed for 1 to 4 weeks.

The ethylene concentration programme may be varied in dependently of the reconditioning programme (for example it may be a constant programme or one that varies based on sprouting that is identified) or dependently (so the ethylene concentration may be varied based on temperature applied by the reconditioning programme or point in time through the reconditioning programme such that it is ramped up or down over time).

The ethylene concentration may be held at a parts-per-million value of 0.1 up to 10. In a preferred embodiment, ethylene concentration is started at approximately 10ppm and reduced over time to 2ppm. Preferably, the concentration is reduced. It may be done in a single step, in weekly steps or in daily steps. Example profiles include 10ppm reducing to 2ppm over the reconditioning period; 10ppm reduced to 2ppm after a predetermined period such as 1 week; 10ppm reducing to 4ppm reducing to 2ppm, a reduction occurring each week; storage at 2ppm for 6 months followed by reconditioning at 1ppm reducing to 0.5ppm and reducing again to 0.1ppm.

The ethylene concentration programme may be dependent on whether the potatoes had previously (before reconditioning) been stored in an ethylene environment.

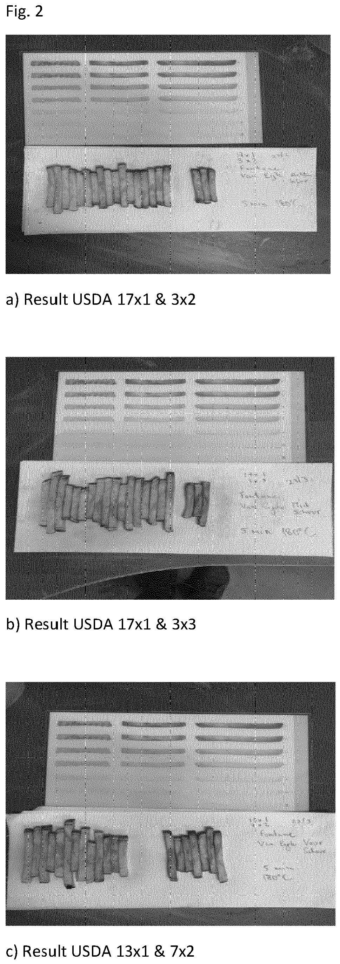

Figure 2 is a series of photographs showing fry colour before reconditioning.

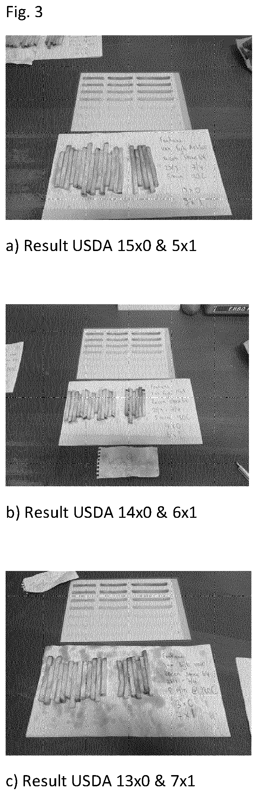

Figure 3 is a series of photographs showing fry colour after reconditioning using the embodiment of Figure 1.

There now follows a description of an experimental trial by the inventors

The goal of the trial was to recondition French fry tubers in a 2-3 week period to improve fry colour and maintain brittle sprouts.

3 trial cells were operated, each 85 cubic meters in size and holding fontane variety of potatoes. The stores holding each trial cell stores were warmed to 12.5°C.

A sample was taken from each cell. Each sample was made up of 25 tubers from the front and the back of the store. The tubers selected were showing signs of sprouting in some degree and have had variable and in some cases poor fry color results.

The first sample was sent for a benchmark Soleye fry colour measurement. Soleye is an optical colour reader used to determine fry colour.

Trial details.
Cell 56
Gradually reducing the ethylene level by 0.5ppm per day from 18% to 4% @ 12 C
Trial dates from 20/3/2020 - 7/4/2020

Cell 60
First 7 days 4ppm and from day 8 reduced to continuous 2ppm @ 12 C
Trial dates from 23/3/2020 - 7/4/2020

Cell 64
Continuous 2ppm @ 12 C
Trial dates from 20/3/2020 - 7/4/2020

The fry colour results in Figures 2 and 3 (prior to reconditioning on 20-4-2020 and after reconditioning on 7-4-2020, respectively) are for the fry colour testing done on samples from store 64. The lower the score, the better the fry colour. The 0 scores in Figure 3 reflecting better fry colour than those of Figure 2.

Following reconditioning using the described method, all tubers maintained brittle sprouts which was the desired outcome.

## Claims

1. A method of reconditioning potatoes comprising:
over a time period of 1 week or more, storing the potatoes in a controlled environment having a temperature of >=10°C and an atmosphere comprising ethylene at a concentration greater than 0.1 ppm.

2. The method of claim 1, further comprising storing potatoes in the controlled environment at a temperature 6-9 Celsius prior to reconditioning and, during reconditioning, warming the controlled environment to 10 to 22 Celsius for 1 to 4 weeks in the presence of ethylene.

3. The method of claim 1 or 2, further comprising controlling ethylene concentration during reconditioning.

4. The method of claim 3, further comprising starting ethylene concentration at 10ppm and dropping concentration over time during reconditioning to 0.1ppm.

5. The method of any preceding claim, wherein the temperature during reconditioning is:
between 10°C and 20°C;
between 12 and 14 °C; or
held at 14 °C.

6. The method of any preceding claim, further comprising varying or maintaining the ethylene concentration in the controlled environment.

7. The method of claim 6, wherein the varying is performed in dependence on one or more of:
sprouting measured or detected in the potatoes; temperature of the controlled environment; and,
a present point in time the potatoes are through a predetermined reconditioning programme.

8. The method of claim 1, wherein the ethylene concentration is held at a parts-per-million value of 0.1 up to 10.

9. The method of claim 1, further comprising reducing the ethylene concentration during reconditioning in a selected one of: a single step, weekly steps, daily steps, hourly steps or steps defined in a predetermined schedule.

10. The method of claim 1 or 9, further comprising starting concentration at a selected one of: 10ppm and reducing to 2ppm over the reconditioning period; or starting concentration at 10ppm and reducing to 4ppm and then reducing to 2ppm the reductions being after the same or differing predetermined periods of time.

11. The method of claim 1, further comprising storing potatoes at 2ppm for 6 months followed by reconditioning in a controlled environment that includes ethylene at 1ppm reducing to 0.5ppm and reducing again to 0.1ppm.

12. The method of any preceding claim, further comprising varying the ethylene concentration programme if the potatoes had, before reconditioning, been stored in an ethylene environment.

13. A system for controlling reconditioning of potatoes in a storage facility comprising:
a temperature sensor and a temperature controller, the temperature sensor being configured to monitor temperature of an atmosphere in the storage facility and communicate the temperature to the temperature controller, the temperature controller being configured to control temperature of the environment according to a predetermined reconditioning programme, the predetermined reconditioning programme maintaining a temperature in the atmosphere of greater than or equal to 10 degrees Celsius for at least one week;
an ethylene concentration sensor and an ethylene concentration controller, the ethylene concentration sensor being configured to detect an ethylene concentration in the atmosphere and communicate the concentration to the ethylene concentration controller, the ethylene concentration controller being configured to control ethylene concentration in the atmosphere according to a predetermined ethylene concentration programme, the predetermined ethylene concentration programme comprising a concentration of ethylene in the atmosphere during the at least one week of at least 0.1 ppm.

14. The system of claim 13, wherein the temperature sensor is configured to monitor temperature of the atmosphere in the storage facility and communicate the temperature to the temperature controller, the temperature controller being configured to control temperature of the environment according to a predetermined reconditioning programme.

15. The system of claim 13 or 14, wherein the ethylene concentration sensor is configured to detect an ethylene concentration in the atmosphere and communicate the concentration to the ethylene concentration controller, the ethylene concentration controller being configured to control ethylene concentration in the atmosphere according to a predetermined ethylene concentration programme.
